# EUROPEAN PATENT SPECIFICATION

(11) **EP 4 031 047 B1**
(45) Date of publication and mention of the grant of the patent: **19.07.2023**
(21) Application number: 20772264.6
(22) Date of filing: 14.09.2020
(51) Int. Cl.: A61B 18/18

(54) **ELECTROSURGICAL APPARATUS FOR TREATING BIOLOGICAL TISSUE WITH MICROWAVE ENERGY**
ELEKTROCHIRURGISCHE VORRICHTUNG ZUR BEHANDLUNG VON BIOLOGISCHEM GEWEBE MIT MIKROWELLENENERGIE
APPAREIL ÉLECTROCHIRURGICAL POUR TRAITER UN TISSU BIOLOGIQUE À L'AIDE D'ÉNERGIE MICRO-ONDE

(30) Priority: 16.09.2019 GB 201913330
(43) Date of publication of application: 27.07.2022
(73) Proprietor: Creo Medical Limited, Chepstow, Monmouthshire NP16 5UH (GB)
(72) Inventor: HANCOCK, Christopher Paul, Monmouthshire NP16 5UH (GB)
(74) Representative: Mewburn Ellis LLP
(86) International application number: PCT/EP2020/075660
(87) International publication number: WO 2021/052913

(56) References cited:
- US-A1- 2006 289 528
- US-A1- 2009 157 070
- US-A1- 2019 029 751

## Description

### FIELD OF THE INVENTION

The invention relates to an electrosurgical apparatus for treating biological tissue with microwave energy, and a method of controlling microwave energy delivered from an electrosurgical instrument into a biological tissue at the distal end of the electrosurgical instrument. In particular, microwave energy is delivered as one or more microwave energy signal pulses, wherein a profile of the one or more microwave energy signal pulses is controlled to cause ablation or coagulation of the biological tissue and to substantially prevent the or each pulse from causing heat to build-up in the electrosurgical instrument. The apparatus may be used transluminally or endoscopically with a scoping device or could be used for open, percutaneous or laparoscopic procedures. The apparatus may be used to treat tissue from within a blood vessel, for example, it could be inserted into the femoral artery.

### BACKGROUND TO THE INVENTION

Electrosurgical systems using microwave energy are disclosed in documents US 2019/029751 A1 and US 2009/157070 A1, for instance. Gaining access to certain tumours for treatment can involve cutting and/or tunnelling through other parts of a patient's body in order to reach a target site where the tumour is located. This can be true for both percutaneous procedures and minimally invasive procedures, such as, laparoscopic or endoscopic procedures. The cutting and/or tunnelling process can cause discomfort to the patient, prolong recovery times, and risk introducing further medical complications.

It is known to use microwave emitting probes to treat various conditions in body tissue, for example, microwave radiation can be used to ablate or coagulate tumours or lesions. For instance, the probe emits microwave energy which agitates water molecules in the surrounding tissue, producing friction and heat, thus inducing cellular death via coagulation necrosis. Using a probe to deliver the microwave energy to target tissue is preferable because the radiating portion can be positioned close to the target site and so a high proportion of power can be transmitted to the target site and a lower proportion is lost to the surrounding healthy tissue. This reduces side effects of treatment as well as increasing efficiency.

Probes can be inserted into tissue via laparoscopic surgery (e.g. using a cannula or tube or inserted directly through the skin if they are rigid enough and sharp enough), open surgery or via channels in the body such as airways. The least invasive method is the use of channels in the body and this reduces strain put on a patient by the procedure. Catheters or scoping devices can be used to help to guide the instrument to the target site.

### SUMMARY OF THE INVENTION

At its most general, the invention provides an electrosurgical apparatus for use in minimally invasive surgical techniques that provides, at a very small scale, a localized microwave field capable of precisely ablating and coagulating tissue from inside a blood vessel (e.g. vein or artery). This is done through suitable selection of geometry and material for a radiating distal tip. Also, the invention delivers microwave energy as one or more microwave energy signal pulses, wherein a profile (e.g. energy, amplitude, peak amplitude, period, duration, duty cycle, ON portion duration, OFF portion duration, etc) of the one or more pulses is selected which causes ablation or coagulation of biological tissue during the one or more pulses but without causing heat to build up in the electrosurgical instrument from pulse to pulse.

For instance, a single pulse may deliver enough energy (e.g. have a high enough peak power, and/or an ON portion with a long enough duration) to cause ablation or coagulation during that single pulse. Additionally or alternatively, a plurality of pulses may combine together to deliver enough energy to cause ablation or coagulation but each individual pulse may not deliver enough energy to cause ablation or coagulation on its own. In this manner, ablation or coagulation is performed.

For instance, heat may not build up in the instrument in a single pulse because the ON portion of that pulse may be so short that dielectric heating of the electrosurgical instrument cannot occur, for example, the ON portion may not be long enough for molecular dipole rotation to generate appreciable heat within the material(s) of the instrument. Additionally or alternatively, heat may not build up in the instrument in a single pulse because the OFF portion of that pulse may be long enough, compared to the ON portion of that pulse, for any heat built up in the instrument during the ON portion to substantially dissipate during the OFF portion. In this manner, unwanted instrument heating is reduced, minimised or avoided which could otherwise cause negative patient outcomes and/or instrument damage.

It is to be understood that either one of coagulation or ablation may be selected by varying the pulse profile (e.g. energy, amplitude, peak amplitude, period, duration, duty cycle, ON portion duration, OFF portion duration, etc). For example, normally, causing ablation of biological tissue takes more energy than causing coagulation to stem a bleed in the same tissue. Therefore, coagulation may be selected by performing fewer doses (e.g. fewer pulses or bursts of pulses) of microwave energy than would be used for performing ablation. Additionally or alternatively, an energy (or peak pulse power or ON portion duration) may be greater when ablation is desired compared to when coagulation is desired.

The apparatus may be used transluminally or endoscopically with a scoping device or could be used for open, percutaneous or laparoscopic procedures. The apparatus may be used to treat tissue from within a blood vessel, for example, it could be inserted into the femoral artery.

According to a first aspect of the invention, there is provided an electrosurgical apparatus for treating biological tissue with microwave energy, the apparatus comprising: a microwave energy signal generator for generating a microwave energy waveform; an electrosurgical instrument arranged to deliver the microwave energy waveform from a distal end thereof for tissue treatment; a controller in communication with the microwave energy signal generator; the microwave energy signal generator being configured to deliver the microwave energy waveform as one or more microwave energy signal pulses, and the controller being configured to control the profile of the one or more microwave energy signal pulses to cause ablation or coagulation of the biological tissue and to substantially prevent the or each pulse from causing heat to build-up in the electrosurgical instrument.

In this manner, the electrosurgical apparatus may be used to perform ablation or coagulation by radiating microwave energy from a distal end of the instrument without building up unwanted heat in other parts of the instrument. Such unwanted built up heat is undesirable because it can cause damage and discomfort to a patient, can delay patient recovery, and lead to medical complications. Also, such unwanted built up heat is undesirable because it can cause damage to the electrosurgical instrument. Further, by selecting a particular pulse profile to avoid unwanted heat building up in the electrosurgical instrument, there is no need to include a separate or integrated cooling mechanism within the apparatus. Where the invention is used to ablate or coagulate tissue from inside a blood vessel, space is at a premium and so there is often not enough room for such cooling mechanisms.

For example, the instrument may include a feed structure (e.g. transmission line or cable) that conveys the microwave energy waveform from the generator to a radiating distal end portion (e.g. antenna) of the instrument. Such unwanted built up heat may cause heating of the feed structure which could generate heat inside healthy regions of a patient along a path from outside the patient's body to a target site within the patient's body, such as a tumour in the patient's body. This unwanted built up heat could cause damage to the healthy regions. Also, this unwanted built up heat could damage the instrument.

The controller may be configured to control the profile of the or each pulse such that an energy of the one or more microwave energy signal pulses is maintained at or above an energy minimum which is set to cause ablation or coagulation of the biological tissue during the one or more microwave energy signal pulses. The energy minimum may be 1 kJ. Since energy is a function of power and time, to meet the energy minimum, the controller may be configured to control the profile of the or each pulse such that a peak power of the or each pulse is maintained at or above a peak power minimum which is set to cause ablation or coagulation of the biological tissue during the one or more microwave energy signal pulses. The peak power minimum may be relatively high for medical applications, such as, 500 W or 1 kW. Additionally or alternatively, to meet the energy minimum, the controller may be configured to control the profile of the or each pulse such that an ON portion of the or each pulse is maintained at or above an ON portion duration minimum which is set to cause ablation or coagulation of the biological tissue during the one or more microwave energy signal pulses. The ON portion duration minimum and the peak power minimum may be set so that the one or more microwave energy signal pulses as a whole deliver at least the energy minimum (e.g. 1 kJ of energy).

The controller may be configured to control the profile of the or each pulse such that a duration of an ON portion of the or each pulse is maintained at or below a first ON portion duration limit which is set to substantially prevent the microwave energy waveform from causing dielectric heating of the electrosurgical instrument during the or each pulse. In this sense, the ON portion may be subject to two conditions: firstly, to be at or above the ON portion duration minimum in order to cause ablation or coagulation and, secondly, to be at or below the first ON portion duration limit in order to avoid dielectric heating of the electrosurgical instrument. For example, where the generator delivers a single pulse, in order to perform ablation or coagulation without causing dielectric heating, the first ON portion duration limit and peak power minimum may be, respectively: 1 s and 1 kW; 0.1 s and 10 kW; 1 ms and 1 MW; and, 0.2 ms and 5 MW. In each of these cases, the energy delivered by the single pulse is at least 1 kJ. In this manner, the ON portion (when constrained by the first ON portion duration limit) may be insufficiently long for appreciable dielectric heating to occur in the electrosurgical instrument. For instance, dielectric heating is caused by molecular dipole rotation within the material(s) of the instrument. That is, at least some molecules which make up the instrument are electric dipoles, meaning that they have a partial positive charge at one end and a partial negative charge at the other, and therefore rotate as they try to align themselves with the alternating electric field of the microwaves. Rotating molecules hit other molecules and put them into motion, thus dispersing energy. This energy, dispersed as molecular rotations, vibrations and/or translations in solids and liquids raises the temperature of the instrument, in a process similar to heat transfer by contact with a hotter body. In this embodiment, the ON portion (when constrained by the first ON portion duration limit) is so short that the molecules are not given sufficient time generate appreciable heating of the instrument in this manner.

The controller may be configured to control the profile of the or each pulse such that a duty cycle of the or each pulse is maintained at or below a duty cycle limit which is set such that heat which the microwave energy waveform causes to be built up in the electrosurgical instrument during an ON portion of that pulse substantially dissipates during an OFF portion of that pulse. Additionally, the controller may be further configured to control the profile of the or each pulse such that the ON portion of the or each pulse is maintained at or below a second ON portion duration limit which is set such that heat which the microwave energy waveform causes to be built up in the electrosurgical instrument during the ON portion of that pulse is substantially dissipated during the OFF portion of that pulse. In an embodiment, the duty cycle limit may be 10%, and/or the second ON portion duration limit may be between 10 µs to 200 µs. In this manner, the electrosurgical instrument may heat up during the ON portion, for example, as a result of dielectric heating. However, the duty cycle (and, possibly, the ON portion duration) is chosen such that substantially all of this heat dissipates during the OFF portion. In this way, heat does not build up from pulse to pulse. Accordingly, the instrument does not generate unwanted built up heat which would otherwise grow to cause negative patient outcomes or damage to the instrument. Stated differently, the pulse profile may be selected so that the heat generated during each pulse is insufficient to cause unwanted damaging heating of the patient or instrument. Since substantially all heat generated during each pulse is dissipated by the end of that pulse, a subsequent pulse will not increase the heating any further, i.e. heat will not build up from pulse to pulse. Also, instead of controlling the ON portion duration (via the second ON portion duration limit), the controller may be configured to control the profile of the or each pulse such that a pulse period of the or each pulse is maintained at or below a pulse period limit which is set such that heat which the microwave energy waveform causes to be built up in the electrosurgical instrument during the ON portion of that pulse is substantially dissipated during the OFF portion of that pulse. The pulse period limit may be 2 ms.

In an embodiment, the microwave energy signal generator is configured to deliver the microwave energy waveform as a plurality of microwave energy signal pulses, and the controller is configured to control the profile of the plurality of microwave energy signal pulses to form a plurality of bursts of pulses, wherein an energy of each burst is maintained at or above the energy minimum. That is, the pulse profile and burst profile are selected such that a burst (as a whole) provides sufficient energy to cause ablation or coagulation, however each individual pulse (on its own) of that burst may not provide sufficient energy to cause ablation or coagulation. Also, each individual pulse (on its own) of that burst is configured to substantially prevent heat to build up in the electrosurgical instrument, for example, because the ON portion is insufficiently long for dielectric heating to occur, or because the duty cycle (and, possibly, ON portion duration or pulse period) is set so that any heat generated during the ON portion substantially dissipates during the OFF portion. In an embodiment, each burst has a burst duty cycle of up to 40%. In an embodiment, each burst has a burst ON portion duration of up to 200 ms. An advantage of arranging the pulses into bursts is that an OFF portion of the bursts further limits thermal heating in the electrosurgical instrument, for example, caused by the microwave energy. This effect is in addition to the above-described thermal heat limiting effect of the specially selected profile of the individual pulses within the bursts.

In an embodiment, the peak power minimum is 1 kW; the duty cycle limit is 10%; and, the first ON portion duration limit is 200 µs. In this example, a 1 kW peak power pulse will be delivered for a ON portion duration of 200 µs, it will then be followed by an OFF portion duration of 1800 µs, and in 1 second there will be 500 pulses of 200 µs duration and the energy delivered into tissue in this 1 second period of time will be 100 J. Therefore, in order to meet the energy minimum of 1 kJ, a dosage of 10 seconds may be required in order to perform ablation or coagulation. In this arrangement, multiple pulses are used to perform ablation or coagulation, however, it is to be understood that in some other embodiments the pulse profile may be varied so that only a single pulse is required (e.g. the peak power minimum could be 5 MW instead of 1 kW). In any case, the profile of each pulse substantially prevents the or each pulse from causing heat to build up in the electrosurgical instrument because (i) dielectric heating is avoided or minimised due to a short ON portion duration of each pulse, and/or (ii) any appreciable heat generated during the ON portion is given time to dissipate during the OFF portion due to a low duty cycle (and, possibly, a short ON portion duration or pulse period).

Whether a single pulse or multiple pulses are used to perform ablation or coagulation without causing heat to build in the electrosurgical instrument, it is important that the instrument (e.g. a coaxial cable and/or a radiating tip portion) is able to withstand high power pulses and, for example, the voltages associated with high power pulses. For example, if 100 kW is to be delivered for 1 second into a 50 ohm load (e.g. tissue load), then the voltage will be about 2,236 V (i.e. SQRT[100,000 x 50]). It is also important that the instrument antenna (e.g. a radiating tip portion) be well impedance matched into the tissue load in order to minimise voltage reflections that may superimpose. Various mechanisms for achieving this impedance match are disclosed in more detail below.

Whether a single pulse or multiple pulses are used to perform ablation or coagulation without causing heat to build in the electrosurgical instrument, it is important that controls be put in place to ensure that a chosen or selected pulse duration (e.g. ON portion duration or pulse period) is not exceeded. This becomes more important as the peak power increases, and so is particularly relevant to embodiments where a single pulse is used to perform ablation or coagulation without causing heat to build in the electrosurgical instrument. For example, to deliver 1 kJ of energy using a 5 MW source would take a duration of 200 µs. Therefore, the controller is operable to accurately enforce this duration of 200 µs and to shut off the microwave energy supply to the instrument at the end of this duration. In an embodiment, the controller may include a shut off circuit that performs this operation. For example, the shut off circuit may include an integrator coupled to a comparator. In operation, the comparator compares an output from the integrator with a preset threshold that corresponds to a given duration (e.g. 200 µs in this case). As the integrator's output accumulates over time this output is compared to the threshold by the comparator and the comparator output changes when the integrator's output reaches the threshold. The generator can be shut off by the controller based on the comparator output. In this way, a mechanism is provided for accurately shutting off the generator at the end of the duration. In an embodiment, the integrator may be clamped, for example, to 5 V.

The electrosurgical instrument may include: a coaxial cable for conveying the microwave energy waveform, the coaxial cable having an inner conductor, an outer conductor, and a first dielectric material separating the inner conductor and the outer conductor; and a radiating tip portion disposed at a distal end of the coaxial cable to receive the microwave energy waveform from the coaxial cable and to radiate a localized microwave field for tissue treatment.

The outer conductor of the coaxial cable may be as physically thick as possible to increase its thermal mass and heat transport capacity. In this way, all or a majority of the heat generated in the cable due to conveying microwave energy can be held within the structure of the cable rather than, for example, being leaked inside the patient. In an embodiment, the outer conductor may be 0.5 mm thick. Additionally or alternatively, heat sinking may be performed at the proximal end of the electrosurgical instrument, such as, in a handle of the electrosurgical instrument. In an embodiment, such heat sinking may be performed by a heat sinking structure (e.g. a solid block of metal, such as, copper) which is connected to the outer conductor of the coaxial cable. Further, the heat sinking structure may include further cooling mechanisms, such as, a cooling fan which directs cooling air onto the heat sinking structure, or a housing or casing which immerses the heat sinking structure in a coolant (e.g. liquid nitrogen).

The radiating tip portion may include a radiopaque structure, e.g. a ring or annular structure on its outer surface, which is visible on a medical imaging system. In this way, the instrument may be visible in spite of having a very small form factor. In an embodiment, at least part of the radiating tip portion (e.g. its distal part) may be made from a high density material, such as ceramic, e.g. zirconia, so that it can be seen under ultrasound imaging, e.g. a hand-held ultrasound imaging system or an endoscopic ultrasound imaging system.

In an embodiment, the radiating tip portion comprises: a dielectric tip, and a distal conductive portion of the inner conductor, which extends longitudinally into the dielectric tip. The dielectric tip may be formed from a second dielectric material that has a dielectric constant greater than the first dielectric material.

In an embodiment, the instrument is thus a coaxial-based device with a dielectric material at its distal end to produce an omnidirectional radiation pattern to create a controllable spherical zone of ablation or coagulation. The geometry of the dielectric radiator determines the shape of the electromagnetic radiation pattern and the tissue affects produced. The distal end of the device is designed to facilitate efficient microwave energy delivery into biological tissue to achieve a localized volume of ablation or coagulation. The resulting localized, thermally induced zone of ablation or coagulation occurs as a result of dielectric heating or a combination of dielectric and thermal conduction. Other antenna geometries may be used. For example, the instrument may include conductive material arranged on an outer surface of the dielectric tip to form a standard microstrip transmission line, a coplanar transmission line, a suspended microstrip line or a leaky co-axial line for delivering the microwave energy into biological tissue. Additionally, the radiating tip portion may include two conductive elements (e.g. discs) separated by an insulator, wherein one conductive element is connected to the inner conductor of the coaxial cable and the other conductive element is connected to the outer conductor of the coaxial cable. Further, the radiating tip portion may include a helical antenna.

The effect of the dielectric tip is to reduce the wavelength of the microwave energy and the structure of the dielectric tip is modelled, using electromagnetic field analysis software to produce better impedance matching and control of the resultant ablation profile based on the small geometry constraints imposed by the dimensions of blood vessels. For example, the outer diameter of the coaxial cable and radiating tip portion may be equal to or less than 1.9 mm, preferably equal to or less than 1.5 mm or even more preferably less than 1mm, This size enables the instrument to fit down the vessel directly or be manipulated by commercially available miniature scoping device instrument channels. This size also enables the instrument to be inserted inside of, and travel within, a blood vessel.

In order to maintain flexibility of the device, the axial length of the dielectric tip is equal to or less than 5 mm, preferably equal to or less than 2 mm. This enables the second dielectric material to be relatively rigid without adversely affecting the flexibility of the instrument, especially at its distal end. In order to shrink the length of the tip by a large enough amount, the dielectric constant of the dielectric may need to be much greater than unity, i.e. 9 or 100, where the wavelength will be shrunk by 3 and 10 respectively,

The microwave energy may be a single spot frequency, e.g. 5.8 GHz or it may be a spot frequency that can be increased or decreased around the spot frequency, e.g. 5.8GHz +/- 100MHz or 2.45GHz +/- 50MHz. This frequency variation can be translated into a change in phase that helps tune or match the microwave energy in the tissue load. In an embodiment, the microwave energy is within a frequency range of 24 GHz to 24.25 GHz (e.g. an ISM band having a centre frequency of 24.125 GHz and a bandwidth of 250 MHz).

The dielectric constant of the second dielectric material may be selected based on the frequency of the microwave energy such that the axial length of the dielectric tip corresponds to a non-negligible fraction of a wavelength of the microwave energy when propagating in the dielectric tip. Herein, a non-negligible fraction may be equal to or greater than 0.05, preferably more than 0.06. This can ensure that the second dielectric material provides a suitable wavelength-shortening effect. In one embodiment, the dielectric constant of the second dielectric material is equal to or greater than 80. For example, titanium dioxide may be used as the second dielectric material. PFTE or any other dielectric that is low loss at the frequency of the microwave energy may be used for the first dielectric material.

The radiating tip portion may be arranged to act as an impedance transformer, for example a quarter wave impedance transformer to match the effective impedance of the antenna to a tissue load impedance. In other words, the geometry of the radiating tip portion is selected so that the effects of the impedance mismatch are invisible when looking into the transmission line prior to the impedance transformer. This may also be considered as being an impedance matching network.

The radiating tip portion may further comprise an intermediate dielectric element surrounding a proximal part of the distal conductive portion and separating the first dielectric material from the dielectric tip, the intermediate dielectric element being formed from a third dielectric material that is different from the second dielectric material. The third dielectric material may be the same as or different from the first dielectric material. The geometry of the intermediate dielectric element can be selected, e.g. based on electromagnetic simulations or the like, to facilitate the impedance matching function discussed above. Again, this may be considered as an impedance matching network.

An embodiment of the instrument may include a handle at the proximal end of the coaxial cable, e.g. to provide an interface to a suitable electrosurgical generator, and a closed ended catheter/sheath for conveying the coaxial cable and radiating tip portion.

The localized microwave field may be substantially spherical, e.g. around the radiating tip portion or it may be elongated, e.g. a cylinder of ablation along the shaft. One advantage of a spherical field shape is that it is rotation invariant, so the orientation of the instrument in the vessel or the instrument channel does not need to be controlled.

An outer sheath may be formed over the radiating tip portion, e.g. to prevent a sharp tip damaging the wall of the vessel or the instrument channel of a scoping device and/or protect the instrument. The dielectric tip may have a geometry that assists manipulation of the instrument within a blood vessel. For example, the distal end of the device may be rounded, e.g. dome-like or hemispherical.

The instrument may further include a temperature sensor at the distal end thereof. The instrument can therefore provide additional feedback about the conditions at the distal end of the instrument. The temperature sensor may be a thermocouple mounted on the outer conductor of the coaxial cable or even on the radiating tip. There may be a plurality of thermocouples positioned around the radiating tip. The thermocouple(s) may be located near a tuning stub or a plurality of stubs, the stub(s) being arranged to filter out a signal having the same frequency as the microwave energy or to force the voltage at or close to the thermocouple to zero or close to zero to ensure that the response (in mV/C or V/C) of the thermocouple is not affected by the microwave signal. To avoid the microwave energy from swamping response signals from the temperature sensor, temperature measurements may also be taken when the microwave energy is off, i.e. in an OFF period of the pulsed operation. Alternatively or additionally, the instrument may include a filtering arrangement for removing noise on the response signal from the temperature sensor caused by the microwave energy, i.e. post filtering may be used to remove the microwave signal (noise) from the measurement signal - a half wavelength filter or a high frequency operational amplifier with a very high common mode rejection ratio (CMRR), e.g. 100dB, may be used to filter out the common mode signal.

The filtering arrangement may include a low pass filter and a common mode injection instrumentation amplifier arranged to remove higher frequency components from the response signal.

The invention may be used in a method of controlling microwave energy delivered from an electrosurgical instrument into a biological tissue at the distal end of the electrosurgical instrument, the method comprising: generating a microwave energy waveform; conveying the microwave energy waveform along a microwave channel to the electrosurgical instrument; delivering the microwave energy waveform from the distal end of the electrosurgical instrument as one or more microwave energy signal pulses; controlling the profile of the one or more microwave energy signal pulses to cause ablation or coagulation of the biological tissue and to substantially prevent the or each pulse from causing heat to build-up in the electrosurgical instrument.

The step of controlling may further include controlling the profile of the or each pulse such that an energy of the one or more microwave energy signal pulses is maintained at or above an energy minimum which is set to cause ablation or coagulation of the biological tissue during the one or more microwave energy signal pulses. The energy minimum may be 1 kJ. In order to meet the energy minimum, the step of controlling may include controlling the profile of the or each pulse such that a peak power of the or each pulse is maintained at or above a peak power minimum which is set to cause ablation or coagulation of the biological tissue during the one or more microwave energy signal pulses. The peak power minimum may be 500 W or 1 kW or more. Additionally or alternatively, to meet the energy minimum, the step of controlling may include controlling the profile of the or each pulse such that an ON portion of the or each pulse is maintained at or above an ON portion duration minimum which is set to cause ablation or coagulation of the biological tissue during the one or more microwave energy signal pulses. The ON portion duration minimum and the peak power minimum may be set so that the one or more microwave energy signal pulses deliver at least the energy minimum (e.g. 1 kJ of energy).

The step of controlling may further include controlling the profile of the or each pulse such that a duration of an ON portion of the or each pulse is maintained at or below a first ON portion duration limit which is set to substantially prevent the microwave energy waveform from causing dielectric heating of the electrosurgical instrument during the or each pulse. For example, where a single pulse is generated, in order to perform ablation or coagulation without causing dielectric heating, the first ON portion duration limit and peak power minimum may be, respectively: 1 s and 1 kW; 0.1 s and 10 kW; 1 ms and 1 MW; and, 0.2 ms and 5 MW. In each of these cases, the energy delivered by the single pulse is at least 1 kJ.

The step of controlling may further include controlling the profile of the or each pulse such that a duty cycle of the or each pulse is maintained at or below a duty cycle limit which is set such that heat which the microwave energy waveform causes to be built up in the electrosurgical instrument during an ON portion of that pulse is substantially dissipated during an OFF portion of that pulse. Also, the step of controlling may further include controlling the profile of the or each pulse such that the ON portion of the or each pulse is maintained at or below a second ON portion duration limit which is set such that heat which the microwave energy waveform causes to be built up in the electrosurgical instrument during the ON portion of that pulse is substantially dissipated during the OFF portion of that pulse. In an embodiment, the duty cycle limit may be 10%, and/or the second ON portion duration limit may be between 10 µs to 200 µs. Further, instead of controlling the ON portion duration, the step of controlling may further include controlling the profile of the or each pulse such that a period of the or each pulse is maintained at or below a pulse period limit which is set such that heat which the microwave energy waveform causes to be built up in the electrosurgical instrument during the ON portion of that pulse is substantially dissipated during the OFF portion of that pulse. The pulse period limit may be 2 ms.

The step of delivering may further include delivering the microwave energy waveform from the distal end of the electrosurgical instrument as a plurality of microwave energy signal pulses; and, the step of controlling may further include controlling the profile of the plurality of microwave energy signal pulses to form a plurality of bursts of pulses, wherein each burst causes ablation or coagulation of the biological tissue. In an embodiment, each burst has a burst duty cycle of up to 40%. In an embodiment, each burst has a burst ON portion duration of up to 200 ms. In an embodiment, each burst delivers at least 1 kJ of energy. However, other burst profiles may be used in other embodiments.

The effects and advantages of the above-described second aspect are as stated above in respect of the first aspect.

The method of controlling microwave energy delivered from an electrosurgical instrument into a biological tissue at the distal end of the electrosurgical instrument may form part of a method of treating a tumour within a patient. For instance, the tumour may attach to (e.g. grow from or branch of off) a patient's blood vessel and the electrosurgical instrument may be inserted through the lumen of the blood vessel to a junction between the blood vessel and the tumour. The electrosurgical instrument may be inserted into the blood vessel percutaneously or via a minimally invasive technique, such as, via a guide catheter or scoping device.

Once inside the blood vessel and at the junction between the blood vessel and the tumour, the pulsed microwave energy can be used to perform various treatments. For example, the microwave energy can be used to treat biological tissue at the junction to cut off a blood supply to the tumour in order to kill the tumour. This technique may involve forming a plug (or solid cell mass) in the tumour at an opening between the tumour and the blood supply so that tumour cells do not leak from the tumour into blood vessel. Additionally or alternatively, the microwave energy can be used to treat biological tissue at the junction to detach the tumour from the blood vessel. This technique may involve forming a plug (or solid cell mass) in the tumour at an opening between the tumour and the blood supply so that tumour cells do not leak from the detached tumour into surrounding parts of the patient's body. This technique also has the effect of killing the tumour by cutting off its blood supply. Additionally or alternatively, the electrosurgical instrument may be inserted through the junction between the blood vessel and the tumour so as to enter inside the tumour. Then, the microwave energy can be used to treat biological tissue inside the tumour to kill the tumour. It is possible that this operation can be performed before the tumour's blood supply is cut off. It is to be understood that in this context treating tissue includes at least one of ablating and coagulating the tissue and, in this way, treating may include the process of elevating the temperature of cancer cells to a level where cell apoptosis occurs and the tumour is destroyed.

Also, the method may involve inserting a catheter (e.g. a guide catheter) through the lumen of the patient's blood vessel to the junction between the blood vessel and the tumour, and then inserting the electrosurgical instrument through the catheter. A distal end of the catheter may be inserted just short of the junction so that the electrosurgical instrument can protrude from the distal end of the catheter and radiate microwave energy directly into cells at a distal end of the instrument.

Herein, microwave frequency may mean a stable fixed frequency in the range 300 MHz to 100 GHz. Preferred spot frequencies for the microwave energy include 915 MHz, 2.45 GHz, 5.8 GHz, 14.5 GHz, 24 GHz and 24.125 GHz.

Herein, the term "conductive" means "electrically conductive" unless the context dictates otherwise.

### BRIEF DESCRIPTION OF DRAWINGS

Examples of the invention are described in more detail below with reference to the accompanying drawings, in which:
Fig. 1A is a schematic diagram of an electrosurgical apparatus with which the present invention can be used;
Fig. 1B is a graphical representation of a microwave energy waveform in accordance with an embodiment;
Fig. 1C is a graphical representation of a microwave energy waveform in accordance with another embodiment;
Fig. 2 is a schematic system diagram of an electrosurgical system in accordance with an embodiment;
Fig. 3 is a longitudinal cross section of an electrosurgical instrument that can be used in embodiments of the invention;
Fig. 4A is a longitudinal cross section of a simulation of the radiation absorption pattern produced by the electrosurgical instrument of Fig. 3;
Fig. 4B is an axial cross section of a simulation of the radiation absorption pattern produced by the electrosurgical instrument of Fig. 3;
Fig. 5 is a longitudinal cross section of an electrosurgical instrument that is another embodiment of the invention; and
Fig. 6 is a longitudinal cross section of a simulation of the radiation absorption pattern produced by the electrosurgical instrument of Fig. 5; and
Fig. 7 is a flow diagram illustrating a method of treating a tumour by controlling microwave energy delivered from an electrosurgical instrument into a biological tissue in accordance with an embodiment.

### DETAILED DESCRIPTION; FURTHER OPTIONS AND PREFERENCES

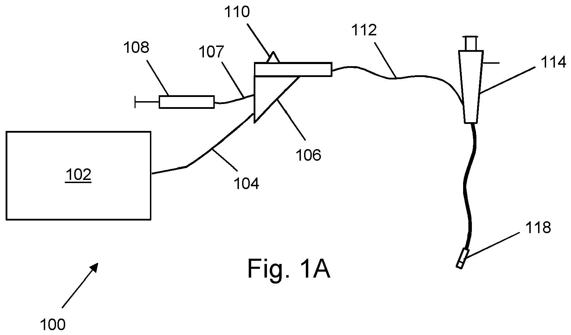

Fig. 1A is a schematic diagram of a complete electrosurgery apparatus 100 that is capable of supplying microwave energy to the distal end of an invasive electrosurgical instrument. The apparatus 100 may also be capable of supplying fluid, e.g. cooling fluid, to the distal end. The apparatus 100 comprises a generator 102 for controllably supplying microwave energy. A suitable generator for this purpose is described in WO 2012/076844. The generator may be arranged to deliver a microwave energy waveform as one or more microwave energy signal pulses. A controller in communication with the generator is configured to control the profile of the one or more microwave energy signal pulses such that, firstly, the pulses cause ablation or coagulation of biological tissue, that is, the one or more pulses have sufficient energy to cause ablation or coagulation. Also, secondly, the controller is configured to control the profile of the one or more microwave energy signal pulses to substantially prevent the or each pulse from causing heat to build-up in the electrosurgical instrument, that is, each pulse is shaped so that it does not leave an appreciable amount of unwanted heat in the instrument once the pulse is complete. A power amplifier of the generator 102 may be specifically selected to enable the generator to deliver such pulses, for example, the power amplifier may be a power amplifier usually used in radar applications. The controller may form part of the generator 102 or may be housed in the same physical unit as the generator 102.

The generator 102 is connected to an interface joint 106 by an interface cable 104. The interface joint 106 may also be connected to receive a fluid supply 107 from a fluid delivery device 108, such as a syringe. If needed, the interface joint 106 can house an instrument control mechanism that is operable by sliding a trigger 110, e.g. to control longitudinal (back and forth) movement of one or more control wires or push rods (not shown). If there is a plurality of control wires, there may be multiple sliding triggers on the interface joint to provide full control. The function of the interface joint 106 is to combine the inputs from the generator 102, fluid delivery device 108 and instrument control mechanism into a single flexible shaft 112, which extends from the distal end of the interface joint 106.

The fluid delivery device 108, the interface cable 104, and the instrument control mechanism are optional.

The flexible shaft 112 is insertable through the entire length of an instrument (working) channel of a scoping device 114 (e.g. a bronchoscope, endoscope, or laparoscope).

The flexible shaft 112 has a distal assembly 118 (not drawn to scale in Fig. 1A) that is shaped to pass through the instrument channel of the scoping device 114 and protrude (e.g. inside the patient) at the distal end of the scoping device's tube. The distal end assembly includes an active tip for delivering or radiating microwave energy into biological tissue. The tip configuration is discussed in more detail below.

The structure of the distal assembly 118 discussed below may be particularly designed for use with a conventional steerable flexible scoping device, whereby the maximum outer diameter of the distal assembly 118 is equal to or less than 2.5 mm, and preferably less than 1.9 mm (and more preferably less than 1.5 mm or even more preferably less than 1mm) and the length of the flexible shaft can be equal to or greater than 1.0 m, e.g. 1.5 m, 2 m, 2.5 m, etc.

The apparatus described above is one way of introducing the instrument. Other techniques are possible. For example, the instrument may also be inserted using a catheter.

The invention seeks to provide an instrument that can travel inside a blood vessel (e.g. vein or artery) and deliver microwave energy to tissue from within the blood vessel, particularly to tissue at a region where a tumour joins to the blood vessel or to tissue inside the tumour itself. For example, the instrument may be used to treat (e.g. ablate or coagulate) tissue at a join or junction between the blood vessel and the tumour to cut-off blood supply to the tumour and, possibly, to detach the tumour from the blood vessel. Additionally or alternatively, the instrument may be used to enter inside the tumour from inside the blood vessel and to deliver microwave energy when inside the tumour. In order for side effects to be reduced and the efficiency of the instrument to be maximised, the transmitting antenna should be located as close to the target tissue as possible. In order to reach the target site, the instrument will need to be guided through the airways and around obstacles. This means that the instrument will ideally be flexible and have a small cross section. Particularly, the instrument should be very flexible near the antenna where it needs to be steered along blood vessels which can be narrow and winding. The size of the antenna part of the instrument should also be reduced where possible to allow the antenna to work properly in small locations and increase flexibility of the instrument when components of the antenna are rigid. The instrument may comprise two coaxial transmission lines arranged in series, with a proximal coaxial transmission line having a greater outer diameter than a distal coaxial transmission line. The outer diameter of the proximal coaxial transmission line may be equal to or greater than 2 mm and the outer diameter of the distal coaxial transmission line may be equal to or less than 1.5 mm, e.g. 1.2 mm. The proximal coaxial transmission line may extend along the majority of the flexible shaft. For example, proximal coaxial transmission line may have a length of 1 m or more and the distal coaxial transmission line may have a length equal to or less than 0.3 m. This arrangement can ensure that more microwave power is delivered into the tissue without the proximal coaxial transmission line getting too hot.

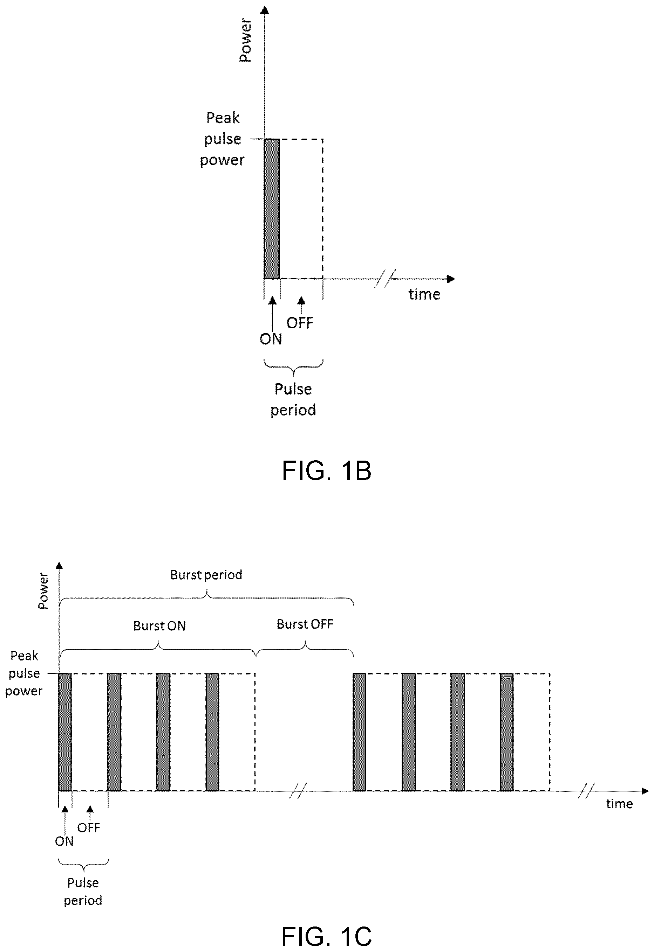

As mentioned above, the generator 102 is controlled (e.g. by a controller) to deliver one or more microwave energy signal pulses which cause ablation or coagulation of biological tissue, wherein the or each pulse is arranged to substantially prevent or avoid causing heat to build-up in the electrosurgical instrument. Two different techniques for avoiding this heat build-up will now be described with reference to Figs. 1B and 1C.

As seen in Fig. 1B, the generator 102 can be controlled to deliver microwave energy as one or more microwave energy signal pulses. Fig. 1B only illustrates a single pulse, but it is to be understood that in some other embodiments multiple pulses may be combined into a series or train of pulses. Specifically, a profile of the one or more microwave energy signal pulses is controlled (i) to cause ablation or coagulation of the biological tissue, and (ii) to substantially prevent the or each pulse from causing heat to build-up in the electrosurgical instrument. Regarding requirement (i), where only a single pulse is provided (e.g. as in Fig. 1B), the pulse profile is controlled so that the energy delivered by this single pulse is at or above an energy minimum which is set to cause ablation or coagulation of the biological tissue during that pulse. This energy minimum may be 1 kJ. Since energy is a function of power and time, in order to achieve this energy minimum, a peak pulse power of the pulse may be maintained at or above a peak power minimum which is set to cause ablation or coagulation of the biological tissue during that pulse. Additionally or alternatively, an ON portion of the pulse may be maintained at or above an ON portion duration minimum which is set to cause ablation or coagulation of the biological tissue during the pulse. On the other hand, where multiple pulses are provided (e.g. a series of the pulse shown in Fig. 1B) the multiple pulses as a whole combine to deliver energy at or above the energy minimum, i.e. enough energy to cause ablation or coagulation, but each individual pulse on its own may not deliver enough energy to cause ablation or coagulation. Therefore, where multiple pulses are used the peak power minimum (and ON portion duration minimum) per pulse may be less than a case where a single pulse is used because the minimum energy requirement can be spread over multiple pulses rather than being provided by a single pulse. Regarding requirement (ii), regardless of whether or not a single pulse or multiple pulses are used, the profile of each pulse is controlled so that a duration of the ON portion of that pulse is maintained at or below a first ON portion duration limit which is set to substantially prevent that pulse from causing dielectric heating of the electrosurgical instrument. Therefore, for a single pulse to satisfy requirements (i) and (ii), the energy delivered by that single pulse must be greater than or equal to the energy minimum to cause ablation or coagulation, but the ON portion of that single pulse must be shorter than the first ON portion duration limit so as to avoid dielectric heating of the instrument. On the other hand, for a series of pulses to satisfy requirements (i) and (ii), the combined energy delivered by the series of pulses must be greater than or equal to the energy minimum so that the series of pulses as a whole cause ablation or coagulation, but the ON portion of each pulse in the series must be shorter than the first ON portion duration limit so as to avoid dielectric heating of the instrument.

In an embodiment, the energy minimum is 1 kJ. Also, the peak power minimum and first ON portion duration limit, respectively, may be any of the following: 1 kW and 1 s; 10 kW and 0.1 s; 1 MW and 1 ms; and, 0.2 ms and 5 MW.

As seen in Fig. 1C, the generator 102 can be controlled to deliver the microwave energy as multiple microwave energy signal pulses. It is to be understood that in some embodiments (e.g. as shown in FIG. 1C), the microwave energy may be delivered as one or more bursts of pulses, i.e. where the multiple pulses are grouped into bursts (or burst periods) having an burst ON portion (with pulse ON portions) and a burst OFF portion (without pulse ON portions). However, in some other embodiments, the microwave energy may be delivered as a single series or train of pulses (which may be analogous to a single burst ON portion, as shown in Fig. 1C). It is to be understood, that each burst and the series/train of pulses can be made up of any number of pulses, including a single pulse. In any case, a profile of each pulse is controlled to keep the combined energy delivered by the multiple microwave energy signal pulses at or above an energy minimum which causes ablation or coagulation of the biological tissue during the multiple microwave energy signal pulses. As before, each pulse may be controlled based on a peak power minimum and/or an ON portion duration minimum to ensure that the multiple pulses deliver at least the energy minimum. For instance, the energy of all the pulses in a single burst (or the complete series/train of pulses) combine together to meet or exceed the energy minimum such that each burst (or the complete series/train of pulses) causes ablation or coagulation, but each individual pulse within that burst (or complete series) may not have sufficient energy to cause ablation or coagulation. Also, the profile of each pulse (i.e. each pulse in the burst or each pulse in the series/train) is controlled to keep a duty cycle of that pulse at or below a duty cycle limit which is set such that heat which the microwave energy waveform causes to be built up in the electrosurgical instrument during an ON portion of that pulse substantially dissipates during an OFF portion of that pulse. It is to be understood that heat dissipation includes the process by which an object that is hotter than other objects is placed in an environment where the heat of the hotter object is transferred to the colder objects and the surrounding environment. Heat dissipation can include conduction, convention and/or radiation.

In an embodiment, instead of or in addition to controlling the duty cycle, the profile of each pulse is controlled to keep the ON portion duration of that pulse at or below a second ON portion duration limit which is set such that heat which the microwave energy waveform causes to be built up in the electrosurgical instrument during the ON portion of that pulse is substantially dissipated during the OFF portion of that pulse. Also, instead of controlling the ON portion duration (via the second ON portion duration limit), the profile of each pulse is controlled such that a pulse period of the or each pulse is maintained at or below a pulse period limit which is set such that heat which the microwave energy waveform causes to be built up in the electrosurgical instrument during the ON portion of that pulse is substantially dissipated during the OFF portion of that pulse.

Accordingly, by controlling the duty cycle (and/or ON portion duration limit or pulse period) a profile of the one or more microwave energy signal pulses is controlled (i) so that the one or more pulses cause ablation or coagulation of the biological tissue, and (ii) to substantially prevent the or each pulse from causing heat to build-up in the electrosurgical instrument. Compared to the embodiment of Fig. 1B, the mechanism by which unwanted heat build-up in the instrument is avoided is different. That is, in Fig. 1B, unwanted heat build-up of the instrument is avoided because the ON portion duration of the or each pulse is below a threshold at which appreciable dielectric heating of the instrument occurs. On the other hand, in Fig. 1C, unwanted heat build-up of the instrument is avoided because the duty cycle (and/or ON portion duration limit or pulse period) is configured so that any unwanted heat which builds up in the instrument during the pulse ON portion (e.g. due to dielectric heating) dissipates during the pulse OFF portion.

In one example, as diagrammatically represented by Fig. 1C, the microwave energy is delivered with a pulse duty cycle of 10% (e.g. a duty cycle limit of 10%). Also, each pulse has a 2 ms pulse period consisting of a 200 µs ON portion and a 1800 µs OFF portion. In this manner, the ON portion duration limit is 200 µs. Hence, the individual pulses have a relatively low duty cycle, i.e. the ON portion duration is small compared to the OFF portion duration. Also, the microwave energy can be delivered such that each ON portion has a power of 1 kW (e.g. a peak power minimum of 1 kW). In this way, each pulse delivers 0.2 J of energy, and in 1 second, 500 pulses are delivered which combine to deliver 100 J of energy. Hence, the ON portion of individual pulses has a high power relative to typical electrosurgical applications (i.e. the pulse has a high peak power), but the average pulse power is much lower (e.g. only 10% of the peak power). The high peak power enables ablation or coagulation to occur, but the lower average power ensures that unwanted equipment and patient heat damage is avoided because heat built-up during each pulse ON portion dissipates during that pulse's OFF portion. Furthermore, the pulses may be arranged into bursts, having a burst period made up from a burst ON portion and a burst OFF portion. In an example, the burst period is 25 ms with a burst ON portion of 10 ms and a burst OFF portion of 15 ms (i.e. a burst duty cycle of 40%). In this way, each burst ON portion contains 5 pulses so that each burst delivers 1 J of energy. However, it is to be understood that in different embodiments, the burst period and burst duty cycle may be different. An advantage of the bursts is that the burst OFF portion further limits unwanted thermal heating in the electrosurgical instrument and patient caused by the microwave energy. It is to be understood, that a single burst may deliver enough energy to cause coagulation, but multiple bursts may be required to deliver enough energy to cause ablation.

In summary, there are many advantages of delivering power as described above with reference to Figs. 1B and 1C.. Firstly, one or more microwave energy signal pulses may be delivered to biological tissue to cause ablation or coagulation in the tissue. Secondly, each pulse may be specially configured so as to avoid causing unwanted heat to build up in the electrosurgical instrument by avoiding dielectric heating of the instrument. Thirdly, each pulse may be specially configured so as to avoid causing unwanted heat to build up in the electrosurgical instrument by ensuing that any unwanted heat generated in the electrosurgical instrument during the ON portion of that pulse is dissipated during the OFF portion of that pulse. As such a result of these advantages, ablation and coagulation can be performed at the treatment site without causing significant temperature rises elsewhere in the patient's body, and without requiring active cooling mechanisms. This is particularly important when the distal assembly and its cable are intended to be located inside a blood vessel, where even small amounts of heating can have a negative impact on patient wellbeing.

The cable for delivering the microwave radiation to the target site should be low-loss, have a small cross-section and be flexible. The cable should be low loss to avoid or reduce heating during treatment and so that there is enough power at the distal end to produce the desired radiation from the antenna.

If the cable is not separated from the body by the use of a sealed scoping device, catheter or other protective sheath, then the cable should be made of, or be coated with, a biologically inert material to avoid unwanted interaction with the body.

A preferred cable type is a coaxial cable which is made up of an inner conductor axially surrounded by a dielectric sheath which is in turn axially surrounded by an outer conductor. The radiating portion in an antenna produced from such a cable may be made up of a section of inner conductor and dielectric sheath which protrudes from the end of the outer conductor of the coaxial cable.

In an embodiment, the outer conductor of the coaxial cable may be as physically thick as possible to increase its thermal mass and heat capacity. In this way, all or a majority of the heat generated in the cable due to conveying microwave energy can be held within the structure of the cable rather than, for example, being leaked inside the patient. In an embodiment, the outer conductor may be 0.5 mm thick.

The invention also seeks to provide an antenna with a well-defined radiation pattern. It is desirable that a practitioner would be able to select an instrument for the treatment of a specific area of tissue, such that the radiation of target tissue is maximised and the radiation of healthy tissue is minimised. For example, in some circumstances it can be desirable to produce a generally spherically symmetric radiation pattern with a substantially uniform power absorption distribution, so that the amount of radiation received by an area of tissue can be more easily controlled by the practitioner.

It is also preferable that the instrument can be operated alongside other instruments to enable practitioners to receive information from the target site. For example, a scoping device may aid the steering of the instruments around obstacles within a patient's body. Other instruments may include a thermometer or camera.

In the following description, unless stated otherwise, the length of a component refers to its dimension in the direction parallel to the longitudinal axis of the coaxial cable.

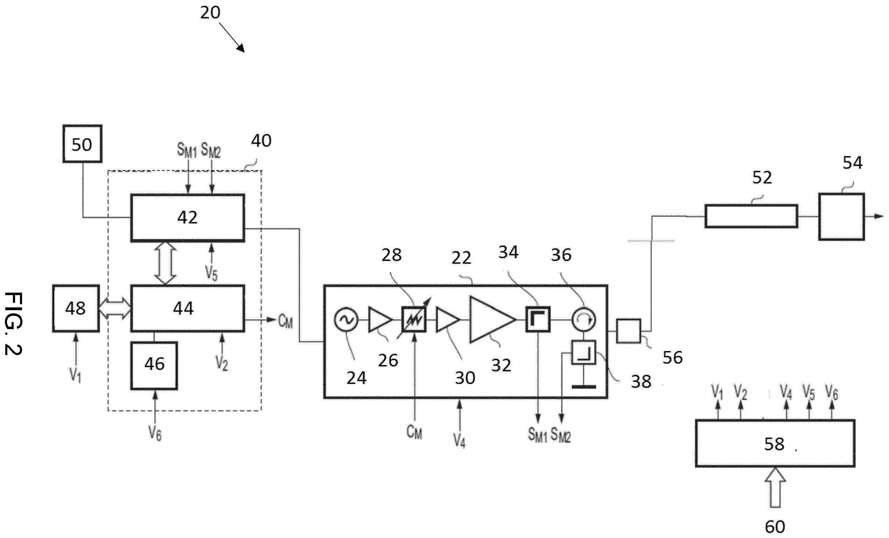

Fig. 2 shows an overall system diagram for an electrosurgical apparatus 20 that is an embodiment of the invention. The apparatus 20 comprises a microwave line-up 22 which forms part of a microwave channel.

The microwave line-up 22 contains components for generating and controlling a microwave frequency electromagnetic signal at a power level suitable for treating (e.g. coagulating or ablating) biological tissue. The microwave line-up 22 of Fig. 2 may form part of the generator 102 of Fig. 1A. In this embodiment, the microwave line-up 22 includes a phase locked oscillator 24, a signal amplifier 26, an adjustable signal attenuator (e.g. an analogue or digital diode attenuator) 28, an amplifier unit (here a driver amplifier 30 and a power amplifier 32), a forward power coupler 34, a circulator 36 and a reflected power coupler 38. The circulator 36 isolates the forward signal from the reflected signal to reduce the unwanted signal components present at the couplers 34, 38, i.e. it increases the directivity of the couplers. Optionally, the microwave line-up 22 includes an impedance matching sub-system having an adjustable impedance. Furthermore, the frequency of the microwave source may be varied around the centre frequency, e.g. 2.45GHz +/-50MHz (2.4GHz to 2.5GHz) or 5.8GHz +/- 100MHz (5.7GHz to 5.9GHz) or 24.125 GHz +/- 125 MHz (24 GHz to 24.25 GHz).

It is to be understood that the power amplifier 32 is configured to enable generation of pulsed waveforms, as described above with reference to Figs. 1B and 1C. For example, the power amplifier 32 may be a high-power pulsed radar RFPA unit, such as those sold by RFHIC Corporation. That is, the inventors have surprisingly discovered that using an amplifier designed for radar applications enables to the aforementioned advantages in medical applications.

The microwave line-up 22 is in communication with a controller 40, which may comprise signal conditioning and general interface circuits 42, a microcontroller 44, and watchdog 46. The controller 40 may form part of the generator 102 of Fig. 1A. The watchdog 46 may monitor a range of potential error conditions, which could result in the apparatus not performing to its intended specification, i.e. the apparatus delivers the wrong dosage of energy into patient tissue due to the output or the treatment time being greater than that demanded by the user. Such a capability is particularly important where a high peak pulse power (e.g. at least 500 W or 1 kW) is being delivered because if this is delivered for longer than intended it could cause damage to the electrosurgical system and the patient. The watchdog 46 comprises a microprocessor that is independent of the microcontroller 44 to ensure that microcontroller is functioning correctly. The watchdog 46 may, for example, monitor the voltage levels from DC power supplies or the timing of pulses determined by the microcontroller 44.

The controller 40 is operable to accurately enforce a preset pulse duration of microwave energy provided to the instrument (e.g. cable 52 and/or probe 54) and to shut off the microwave energy supply to the instrument at the end of this pulse duration. In an embodiment, the controller 40 may include a shut-off circuit that performs this operation. For example, the shut off circuit may include an integrator coupled to a comparator. In operation, the comparator compares an output from the integrator with a preset threshold that corresponds to a given pulse duration. As the integrator's output accumulates over time this output is compared to the threshold by the comparator and the comparator output changes when the integrator's output reaches the threshold. The microwave supply can be shut off by the controller 40 based on the comparator output. In this way, a mechanism is provided for accurately shutting off the microwave supply at the end of the pulse duration. In an embodiment, the integrator may be clamped, for example, to 5 V. In an embodiment, the shut-off circuit may be part of the watchdog 46.

The controller 40 is arranged to communicate control signals to the components in the microwave line-up 22. In this embodiment, the microprocessor 44 is programmed to output a microwave control signal C_{M} for the adjustable signal attenuator 28. This control signal is used to set the energy delivery profile of the microwave EM radiation output from the microwave line-up 22. In particular, the adjustable signal attenuator 28 is capable of controlling the power level of the output radiation. Moreover, the adjustable signal attenuator 28 may include switching circuitry capable of setting the waveform (e.g. pulse energy, pulse peak power, pulse period, pulse duty cycle, pulse ON portion, pulse OFF portion, burst energy, burst period, burst duty cycle, burst ON portion, etc.) of the output radiation. Therefore, the controller 40 can use the control signal C_{M} to cause the system 20 to deliver a microwave energy waveform according to Fig. 1B or 1C discussed above.

The microprocessor 44 may be programmed to output the microwave control signal C_{M} based on forward and reflected power couplers 34, 38. In this embodiment, the microwave generator may be controlled by measurement of phase information only, which can be obtained from the microwave channel (from sampled forward and reflected power information). The forward power coupler 34 outputs a signal S_{M1} indicative of the forward power level and the reflected power coupler 38 outputs a signal S_{M2} indicative of the reflected power level. The signals S_{M1}, S_{M2} from the forward and reflected power couplers 34, 38 are communicated to the signal conditioning and general interface circuits 42, where they are adapted to a form suitable for passing to the microprocessor 44.

It is to be understood that outputting the microwave control signal C_{M} based on forward and reflected power couplers 34, 38 is optional. For example, in some other embodiments, the microprocessor 44 may be programmed to output the microwave control signal C_{M} in an open loop manner, i.e. without consideration of the forward and reflected power.

A user interface 48, e.g. touch screen panel, keyboard, LED/LCD display, membrane keypad, footswitch or the like, communicates with the controller 40 to provide information about treatment to the user (e.g. surgeon) and permit various aspects of treatment (e.g. the amount of energy delivered to the patient, or the profile of energy delivery) to be manually selected or controlled, e.g. via suitable user commands. The apparatus may be operated using a conventional footswitch 50, which is also connected to the controller 40. In an embodiment, the user interface 48 and the foot switch 50 may form part of the controller 40.

The microwave signals produced by the microwave line-up 22 are input to a cable assembly 52 (e.g. a coaxial cable) an onwards to a probe 54 (or applicator). The probe 54 of Fig. 2 may provide the distal assembly 118 of Fig. 1A. The cable assembly 52 allows energy at microwave frequencies to be transmitted to the probe 54, from which it is delivered (e.g. radiated) into the biological tissue of a patient. Example structures of the probe 54 are discussed below.

The cable assembly 52 also permits reflected energy, which returns from the probe 54, to pass into the microwave line-up 22, e.g. to be detected by the detectors contained therein. The apparatus may include a high pass filter 56 on the microwave channel, so that only a reflected microwave signal enters the microwave line-up 22.

Finally, the apparatus includes a power supply unit 58 which receives power from an external source 60 (e.g. mains power) and transforms it into DC power supply signals V₁, V₂, V₄, V₅, and V₆ for the components in the apparatus. Thus, the user interface receives a power signal V₁, the microprocessor 110 receives a power signal V₃, the microwave line-up 22 receives a power signal V₄, the signal conditioning and general interface circuits 42 receive a power signal V₅, and the watchdog 46 receives a power signal V₆.

As mentioned above, a suitable generator for controllably supplying microwave energy is described in WO 2012/076844 and, therefore, the apparatus 20 presents only one possible implementation for generating microwave energy and the other implementations described in WO 2012/076844 are also applicable. However, it is to be understood that the power amplifier of the generator must be capable of generating waveforms in accordance with the present invention (e.g. as per Figs. 1B or 1C).

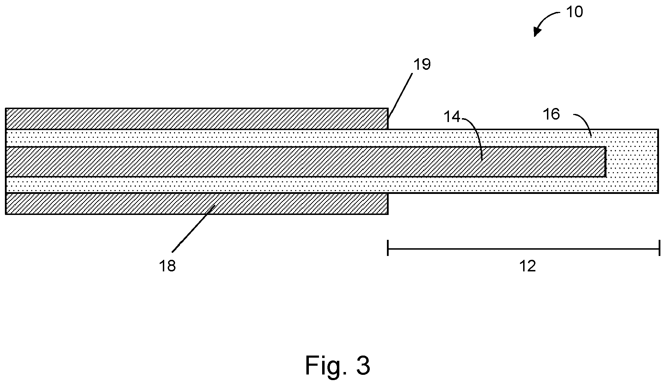

Fig. 3 is a longitudinal cross section taken along the axis of a coaxial cable which forms an electrosurgical instrument or tissue ablation antenna 10. The tissue ablation antenna 10 may include the distal assembly 118 of Fig. 1A, or the probe 54 and cable 52 of Fig. 2. The tissue ablation antenna 10 may therefore be used to deliver a microwave energy waveform according to Figs. 1B and 1C discussed above. The tissue ablation antenna 10 comprises a radiating portion 12. The inner conductor 14 is radially surrounded by a dielectric sheath 16 which is in turn radially surrounded by the outer conductor 18. The inner conductor 14 and the insulating sheath 16 extend beyond a distal end 19 of the outer conductor 18 and the protruding section of inner conductor and insulating sheath forms the radiating portion 12. In this example, the inner conductor 14 is shorter than the insulating sheath 16 so that the end of the insulating sheath 16 forms a cap over the inner conductor 14.

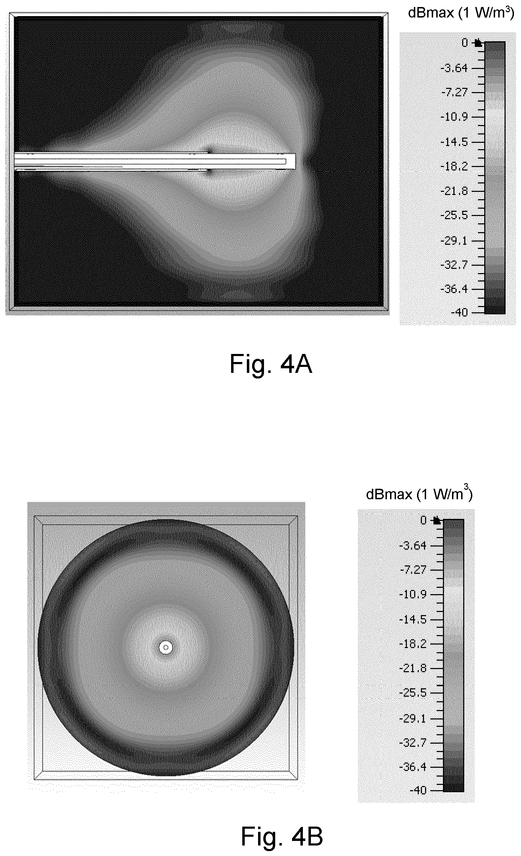

Figs. 4A and 4B show longitudinal and axial cross-sections respectively of a radiation pattern simulation for the antenna 10 shown in Fig. 3. It can be seen that the pattern covers an elongated region near the end of the outer conductor 18. It is axially symmetric and is generally strongest at the distal end 19 of the outer conductor 18.

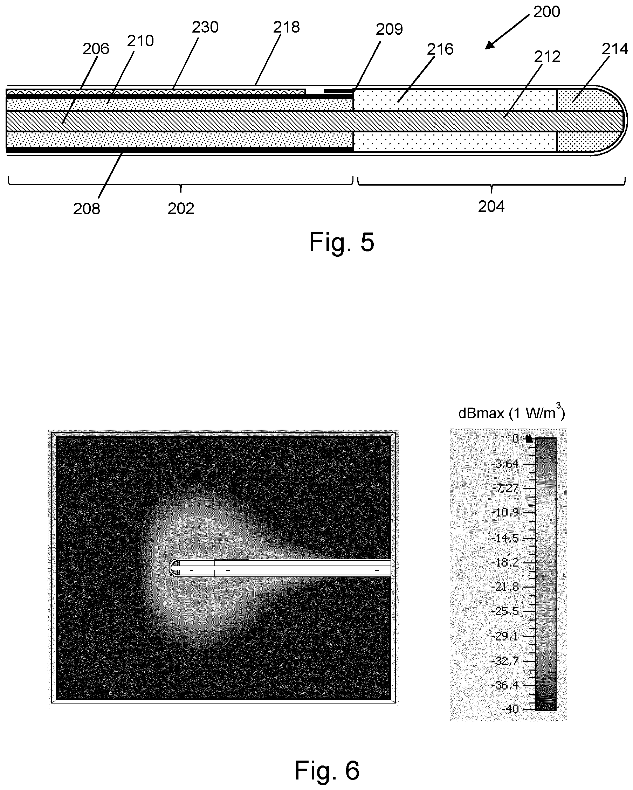

Fig. 5 is a cross-sectional view of the distal end of an electrosurgical instrument 200 that is an embodiment of the invention. The electrosurgical instrument 200 may include the distal assembly 118 of Fig. 1A, or the probe 54 and cable 52 of Fig. 2. The electrosurgical instrument 200 may therefore be used to deliver a microwave energy waveform according to Figs. 1B and 1C discussed above. The electrosurgical instrument 200 comprises a coaxial cable 202 that is connected at its proximal end to an electrosurgical generator (not shown) in order to convey microwave energy. The coaxial cable 202 comprises an inner conductor 206, which is separated from an outer conductor 208 by a first dielectric material 210. The coaxial cable 202 is preferably low loss for microwave energy. A choke (not shown) may be provided on the coaxial cable to inhibit back propagation of microwave energy reflected from the distal end and therefore limit backward heating along the device.

The device may include a temperature sensor at the distal end. For example, in Fig. 5 a thermocouple 230 is mounted on the outer conductor to transmit a signal back to the proximal end that is indicative of temperature at the distal end of the instrument.

Other techniques for temperature monitoring can be used. For example, one or more micromechanical structures whose physical configuration is sensitive to temperature may be mounted in the distal portion of the device, e.g. in or on the outer sheath discussed below. These structures can be interfaced with an optical fibre, whereby changes in a reflected signal caused by movement of the structure can be indicative of temperature changes.

The coaxial cable 202 terminates at its distal end with a radiating tip section 204. In this embodiment, the radiating tip section 204 comprises a distal conductive section 212 of the inner conductor 206 that extends beyond a distal end 209 of the outer conductor 208. The distal conductive section 212 is surrounded at its distal end by a dielectric tip 214 formed from a second dielectric material, which is different from the first dielectric material 210. The length of the dielectric tip 214 is shorter than the length of the distal conductive section 212. An intermediate dielectric sleeve 216 surrounds the distal conductive section 212 between the distal end of the coaxial cable 202 and the proximal end of the dielectric tip 214. The intermediate dielectric sleeve 216 is formed from a third dielectric material, which is different from the second dielectric material but which may be the same as the first dielectric material 210.

In this embodiment, the coaxial cable 202 and radiating tip section 204 have an outer sheath 218 formed over their outermost surfaces. The outer sheath 218 may be formed from a biocompatible material. The outer sheath 218 has a thickness that is small enough to ensure that it does not significantly interfere with the microwave energy radiated by the radiating tip section 204 (i.e. radiating pattern and return loss). In an embodiment, the sheath is made from PTFE, although other materials are also appropriate. The thickness of the wall of the sheath is selected to withstand breakdown voltages equal to or greater than 200 kV/m.

The purpose of the dielectric tip 214 is to alter the shape of the radiated energy. The second dielectric material is selected to reduce the wavelength of the microwave energy, which results in the radiated energy exhibiting a more spherical radiation pattern. To do this, the second dielectric material preferably has a large dielectric constant (relative permittivity *εᵣ*). The dielectric constant of the second dielectric material is preferably chosen to enable the length of the dielectric tip 214 to be minimised whilst still constituting a non-negligible portion of a wavelength of the microwave energy when it propagates through the second dielectric material. It is desirable for the dielectric tip 214 to be as short as possible in order to retain flexibility in the device, especially if the second dielectric material is rigid. In an embodiment, the dielectric tip 214 may have a length equal to or less than 2 mm. The dielectric constant of the second dielectric material may be greater than 80, and is preferably 100 or more at the frequency of the microwave energy. The second dielectric material may be TiO₂ (titanium dioxide).

The wavelength of radiation in a material becomes shorter as the dielectric constant of the material increases. Therefore a dielectric tip 214 with a greater dielectric constant will have a greater effect on the radiation pattern. The larger the dielectric constant, the smaller the dielectric tip 214 can be while still having a substantial effect on the shape of the radiation pattern. Using a dielectric tip 214 with a large dielectric constant means that the antenna can be made small and so the instrument can remain flexible. For example the dielectric constant in TiO₂ is around 100. The wavelength of microwave radiation having a frequency of 5.8 GHz is about 6 mm in TiO₂ compared to around 36 mm in PTFE (which may be the material used for the first and/or third dielectric materials). A noticeable effect on the shape of the radiation pattern can be produced in this arrangement with a dielectric tip 214 of approximately 1 mm. As the dielectric tip 214 is short, it can be made from a rigid material whilst still maintaining flexibility of the antenna as a whole.

The dielectric tip 214 may have any suitable distal shape. In Fig. 5 it has a dome shape, but this is not necessarily essential. For example, it may be cylindrical, conical, etc. However, a smooth dome shape may be preferred because it increases the mobility of the antenna as it is manoeuvred through small channels (e.g. inside blood vessels). The dielectric tip 214 may be coated with a non-stick material such as Parylene C or Parylene D, or PFTE to prevent the tissue from sticking to the instrument. The whole instrument can be coated in this way.

The properties of the intermediate dielectric sleeve 216 are preferably chosen (e.g. through simulation or the like) so that the radiating tip section 204 forms a quarter wave impedance transformer for matching the input impedance of the generator into a biological tissue load in contact with the radiating tip section 204.

A longitudinal cross section of a simulation of the absorption pattern of an antenna having the configuration shown in Fig. 5 is shown in Fig. 6. The pattern produced is more uniform and more spherical than the pattern shown in Figs. 4A and 4B. The pattern in Fig. 6 is axially symmetric and more of the radiation is concentrated around the radiating portion rather than spreading down the cable as occurs in Figs. 4A and 4B. This means that, when in use, an area of tissue may be radiated more uniformly, meaning there is less chance of damage to healthy tissue. The radiation is also less spread out, allowing the practitioner to more accurately radiate target tissue and reduce radiation of or damage to healthy tissue. The pear drop shape of radiation pattern shown in Fig. 6 may also be particularly useful for treating fibroids.

During treatment, the surrounding tissue absorbs the radiated energy. The volume of tissue into which the energy is delivered depends on the frequency of the microwave energy.

It is to be understood that in some other embodiments the structure of the radiating tip portion 204 may be different and may not include a dielectric tip 214. For example, the radiating tip portion may include two conductive elements (e.g. disks) separated by an insulator, wherein one of the conductive elements is connected to the inner conductor 206 of the coaxial cable 202 and the other one of the conductive elements is connected to the outer conductor 208 of the coaxial cable 202. Alternatively, the radiating tip portion may include a helical antenna. For example, an insulator or dielectric element may have two helical electrodes arranged on its surface, wherein one of the helical electrodes is connected to the inner conductor 206 of the coaxial cable 202 and the other of the helical electrodes is connected to the outer conductor 208 of the coaxial cable 202. Alternatively, other radiating tip portion structures may include slotted antennas.

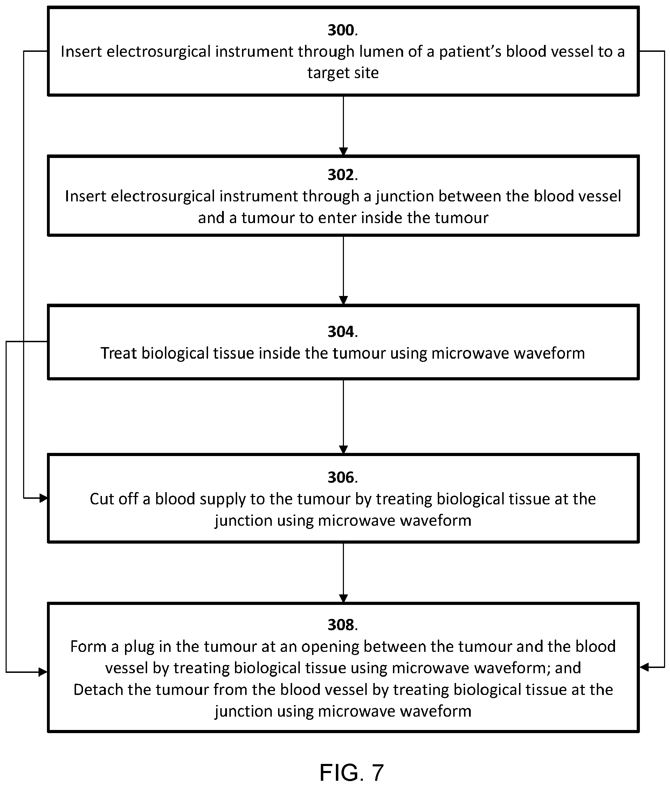

Fig. 7 illustrates a method of controlling microwave energy delivered from an electrosurgical instrument into a biological tissue at the distal end of the electrosurgical instrument, an accordance with an embodiment. The method may be implemented using the electrosurgical apparatuses described above with reference to Figs. 1A, 2, 3 and 5. Furthermore, the method can be used to treat tumours which are joined to blood vessels.

The method begins at block 300. At block 300, an electrosurgical instrument is inserted into a blood vessel (e.g. vein or artery) within a patient. For example, the electrosurgical instrument may be as shown in Figs. 3 or 5. The instrument is moved through the blood vessel until it reaches a target site. In an embodiment, the target site is at or near to where a tumour joins to the blood vessel. The tumour may be connected to or may grow from (e.g. branch off of) the blood vessel such that the tumour receives a blood supply from the blood vessel. In another embodiment, the target site may be elsewhere inside the blood vessel. Once the electrosurgical instrument is at the target site, processing flows to block 302.

At block 302, optionally, the electrosurgical instrument is pushed through a junction between the blood vessel and the tumour so that the distal end of the electrosurgical instrument enters inside the tumour (e.g. a centre of the tumour). At block 304, the electrosurgical instrument is activated to radiate microwave energy from the distal end (e.g. as per the above-described pulse profile of Fig. 1B or 1C) in order to treat (e.g. ablate or coagulate) biological tissue inside the tumour. In this way, the tumour may be destroyed or killed from the inside. Further details of what constitutes activation of the electrosurgical instrument are included below.

In addition to blocks 302 and 304, or as an alternative to blocks 302 and 304, at block 306, the electrosurgical instrument is positioned at the junction between the blood vessel and the tumour (i.e. the target site) and is activated to treat (e.g. ablate or coagulate) the biological tissue which forms the junction. In this way, the biological tissue at the junction is destroyed so as to cut-off a blood supply to starve the tumour of blood and to kill the tumour.

In addition to blocks 302 to 306, or as an alternative to blocks 302 to 306, at block 308, the electrosurgical instrument is positioned at the junction between the blood vessel and the tumour (i.e. the target site) and is activated to treat (e.g. coagulate) the biological tissue at an opening between the tumour and the blood vessel so as to form a plug (e.g. a solid mass of cells) in the tumour which seals the opening shut. Next, the electrosurgical instrument is activated to treat (e.g. ablate) the biological tissue at the junction to detach the tumour from the blood vessel. A consequence of detaching the tumour from the blood vessel is that the tumour's blood supply is cut-off thereby starving the tumour of blood and killing the tumour. The detached tumour may be left to travel around the patient's body because, since its blood supply has been cut-off, the detached tumour can no longer grow or spread around the body. It is noted that the act of forming a plug which seals shut the tumour opening where it once joined to the blood vessel avoids tumour cells leaking out of the detached tumour.

Preferably, the method includes each of blocks 300 to 308. However, alternatively, the method may involve only blocks 300, 302 and 304, or only blocks 300 and 306, or only blocks 300 and 308, or only blocks 300, 306 and 308, or only blocks 300, 302, 304 and 308. This is indicated on Fig. 7 by various arrows between the blocks.

Also, block 300 may involve inserting a guiding device (e.g. a guide catheter or a scoping device) through the lumen of the patient's blood vessel and positioning a distal end of the catheter at or near to the target site. Then, the electrosurgical instrument may be positioned at or near to the target site by inserting the instrument through a lumen of the guiding device. In an embodiment, the guiding device may be stopped before reaching the target site, so that the electrosurgical instrument can protrude from an opening at a distal end of the guiding device to directly reach the target site.

It is to be understood that the process of activating the electrosurgical instrument to treat biological tissue involves the operations performed by, for example, the electrosurgical apparatus of Figs. 1A, 2, 3 and 5, as discussed above. That is, the electrosurgical instrument may be controlled to deliver a microwave energy waveform according to Fig. 1B or 1C, discussed above. Generally, these operations include: generating a microwave energy waveform; conveying the microwave energy waveform along a microwave channel to the electrosurgical instrument; delivering the microwave energy waveform into biological tissue from the distal end of the electrosurgical instrument as one or more microwave energy signal pulses; controlling the profile of the one or more microwave energy signal pulses to cause ablation or coagulation of the biological tissue and to substantially prevent the or each pulse from causing heat to build-up in the electrosurgical instrument. In this way, the profile of the one or more microwave energy signal pulses is controlled to cause ablation or coagulation of the biological tissue but each pulse is arranged such that heat does not to build-up in the electrosurgical instrument. A more detailed explanation of the one or more microwave energy signal pulses in accordance with different embodiments are described above with reference to Figs. 1B and 1C.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many variations will be apparent to those skilled in the art when given this disclosure. Accordingly, the exemplary embodiments of the invention set forth above are considered to be illustrative and not limiting. Various changes to the described embodiments may be made without departing from the scope of the invention

For the avoidance of any doubt, any theoretical explanations provided herein are provided for the purposes of improving the understanding of a reader. The inventors do not wish to be bound by any of these theoretical explanations.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

## Claims

1. An electrosurgical apparatus for treating biological tissue with microwave energy, the apparatus comprising:
a microwave energy signal generator (102) for generating a microwave energy waveform;
an electrosurgical instrument (54) arranged to deliver the microwave energy waveform from a distal end thereof for tissue treatment;
a controller (40) in communication with the microwave energy signal generator
the microwave energy signal generator being configured to deliver the microwave energy waveform as one microwave energy signal pulse, and
the controller being configured to control the profile of the one microwave energy signal pulse to cause ablation or coagulation of the biological tissue and to substantially prevent the one pulse from causing heat to build-up in the electrosurgical instrument,
wherein the controller is configured to control the profile of the one pulse such that a peak power of the one pulse is maintained at or above a peak power minimum which is set to cause ablation or coagulation of the biological tissue during the one microwave energy signal pulse, the peak power minimum being 500 W, and
wherein at least one of the following applies:
(a) the controller is configured to control the profile of the one pulse such that a duration of an ON portion of the one pulse is maintained at or below an ON portion duration limit which is set to substantially prevent the microwave energy waveform from causing dielectric heating of the electrosurgical instrument during the one pulse, the ON portion duration limit being 1 s;
(b) the controller is configured to control the profile of the one pulse such that a duty cycle of the one pulse is maintained at or below a duty cycle limit which is set such that heat which the microwave energy waveform causes to be built up in the electrosurgical instrument during an ON portion of the one pulse substantially dissipates during an OFF portion of the one pulse.

2. The electrosurgical apparatus of claim 1, wherein the controller is configured to control the profile of the one pulse such that an energy of the one microwave energy signal pulse is maintained at or above an energy minimum which is set to cause ablation or coagulation of the biological tissue during the one microwave energy signal pulse.

3. The electrosurgical apparatus of claim 2, wherein the energy minimum is 1 kJ.

4. The electrosurgical apparatus of any preceding claim, wherein the peak power minimum is any one of the following: 1 kW, 10 kW, 1 MW, 5 MW.

5. The electrosurgical apparatus of any preceding claim, wherein the ON portion duration limit is any one of the following: 0.1 s, 1 ms, 0.2 ms.

6. The electrosurgical apparatus of any preceding claim, wherein at least one of the following applies:
(a) the duty cycle limit is 10%,
(b) the ON portion has a duration of between 10 µs to 200 µs.

7. The electrosurgical apparatus of any one of claims 3 to 6 when dependent on claim 2, wherein:
the microwave energy signal generator is configured to deliver the microwave energy waveform as a plurality of microwave energy signal pulses, and
the controller is configured to control the profile of the plurality of microwave energy signal pulses to form a plurality of bursts of pulses, wherein an energy of each burst is maintained at or above the energy minimum.

8. The electrosurgical apparatus of claim 7, wherein at least one of the following applies:
(a) each burst has a burst duty cycle of up to 40%,
(b) each burst has a burst ON portion duration of up to 200 ms.

9. The electrosurgical apparatus of any preceding claim, wherein the electrosurgical instrument comprises:
a coaxial cable for conveying the microwave energy waveform, the coaxial cable having an inner conductor, an outer conductor, and a first dielectric material (16) separating the inner conductor (14) and the outer conductor (18); and
a radiating tip portion (12, 204) disposed at a distal end of the coaxial cable to receive the microwave energy waveform from the coaxial cable and to radiate a localized microwave field for tissue treatment

10. The electrosurgical apparatus of claim 9, wherein the radiating tip portion (12, 204) comprises:
a dielectric tip (214), and
a distal conductive portion of the inner conductor, said distal conductive portion extending longitudinally into the dielectric tip.

11. The electrosurgical apparatus according to claim 10, wherein the outer diameter of the coaxial cable and radiating tip portion is equal to or less than 2.5 mm.

12. The electrosurgical apparatus of claim 9, wherein the radiating tip portion comprises two conductive elements separated by an insulator, and wherein one conductive element is connected to the inner conductor of the coaxial cable and the other conductive element is connected to the outer conductor of the coaxial cable.

13. The electrosurgical apparatus of claim 9, wherein the radiating tip portion comprises a helical antenna.

14. The electrosurgical apparatus of any of claims 9 to 13, wherein the radiating tip portion is arranged to act as a quarter wave impedance transformer to match an input impedance to a tissue load impedance.

## Patentansprüche

1. Elektrochirurgische Vorrichtung zur Behandlung von biologischem Gewebe mit Mikrowellenenergie, wobei die Vorrichtung Folgendes umfasst:
einen Mikrowellenenergie-Signalgenerator (102) zum Erzeugen einer Mikrowellenenergie-Wellenform;
ein elektrochirurgisches Instrument (54), das angeordnet ist, um die Mikrowellenenergie-Wellenform von einem distalen Ende desselben zur Gewebsbehandlung abzugeben;
eine Steuerung (40) in Kommunikation mit dem Mikrowellenenergie-Signalgenerator, wobei der Mikrowellenenergie-Signalgenerator ausgelegt ist, um die Mikrowellenenergie-Wellenform als einen Mikrowellenenergie-Signalimpuls abzugeben, und
die Steuerung ausgelegt ist, um das Profil des einen Mikrowellenenergie-Signalimpulses zu steuern, um eine Ablation oder Koagulation des biologischen Gewebes herbeizuführen und im Wesentlichen zu verhindern, dass der eine Impuls einen Wärmestau in dem elektrochirurgischen Instrument herbeiführt,
wobei die Steuerung ausgelegt ist, um das Profil des einen Impulses zu steuern, sodass eine Peakleistung des einen Impulses bei oder über einem Peakleistungsminimum gehalten wird, die festgelegt ist, um eine Ablation oder Koagulation des biologischen Gewebes während des einen Mikrowellenenergie-Signalimpulses herbeizuführen, wobei das Peakleistungsminimum 500 W beträgt, und
wobei zumindest eines des Folgenden gilt:
(a) die Steuerung ist ausgelegt, um das Profil des einen Impulses so zu steuern, dass eine Dauer eines EIN-Abschnitts des einen Impulses bei oder unterhalb einer EIN-Abschnittsdauergrenze gehalten wird, die festgelegt ist, um die Mikrowellenenergiewellenform im Wesentlichen daran zu hindern, ein dielektrisches Erhitzen des elektrochirurgischen Instruments während des einen Impulses herbeizuführen, wobei die EIN-Abschnittsdauergrenze 1 s beträgt;
(b) die Steuerung ist ausgelegt, um das Profil des einen Impulses so zu steuern, dass ein Tastverhältnis des einen Impulses bei oder unter einer Tastverhältnisgrenze gehalten wird, die so festgelegt ist, dass der Wärmestau, der von der Mikrowellenenergie-Wellenform in dem elektrochirurgischen Instrument während eines EIN-Abschnitts des einen Impulses herbeigeführt wird, während eines AUS-Abschnitts des einen Impulses im Wesentlichen abgebaut wird.

2. Elektrochirurgische Vorrichtung nach Anspruch 1, wobei die Steuerung ausgelegt ist, um das Profil des einen Impulses so zu steuern, dass eine Energie des einen Mikrowellenenergie-Signalimpulses bei oder über einem Energieminimum gehalten wird, das festgelegt ist, um eine Ablation oder Koagulation des biologischen Gewebes während des einen Mikrowellenenergie-Signalimpulses herbeizuführen.

3. Elektrochirurgische Vorrichtung nach Anspruch 2, wobei das Energieminimum 1 kJ beträgt.

4. Elektrochirurgische Vorrichtung nach einem der vorangegangenen Ansprüche, wobei das Peakleistungsminimum ein beliebiges der folgenden ist: 1 kW, 10 kW, 1 MW, 5 MW.

5. Elektrochirurgische Vorrichtung nach einem der vorangegangenen Ansprüche, wobei die EIN-Abschnittsdauergrenze eine beliebige der folgenden ist: 0,1 s, 1 ms, 0,2 ms.

6. Elektrochirurgische Vorrichtung nach einem der vorangegangenen Ansprüche, wobei zumindest eines des Folgenden gilt:
(a) die Tastverhältnisgrenze beträgt 10 %,
(b) der EIN-Abschnitt weist eine Dauer von 10 µs bis 200 µs auf.

7. Elektrochirurgische Vorrichtung nach einem der Ansprüche 3 bis 6 in Abhängigkeit von Anspruch 2, wobei:
der Mikrowellen-Signalgenerator ausgelegt ist, um die Mikrowellenenergie-Wellenform als Vielzahl von Mikrowellenenergie-Signalimpulsen abzugeben, und
die Steuerung ausgebildet ist, um das Profil der Vielzahl von Mikrowellenenergie-Signalimpulsen zu steuern, um eine Vielzahl von Impulsabfolgen auszubilden, wobei eine Energie jeder Abfolge bei oder über dem Energieminimum gehalten wird.

8. Elektrochirurgische Vorrichtung nach Anspruch 7, wobei zumindest eines des Folgenden gilt:
(a) jede Abfolge weist ein Abfolgetastverhältnis von bis zu 40 % auf,
(b) jede Abfolge weist eine Abfolge-EIN-Abschnittsdauer von bis zu 200 ms auf.

9. Elektrochirurgische Vorrichtung nach einem der vorangegangenen Ansprüche, wobei das elektrochirurgische Instrument Folgendes umfasst:
ein Koaxialkabel zum Übertragen der Mikrowellenenergie-Wellenform, wobei das Koaxialkabel einen Innenleiter, einen Außenleiter und ein erstes dielektrisches Material (16), das den Innenleiter (14) und den Außenleiter (18) trennt, aufweist; und
einen Strahlungsspitzenabschnitt (12, 204), der an einem distalen Ende des Koaxialkabels angeordnet ist, um die Mikrowellenenergie-Wellenform von dem Koaxialkabel aufzunehmen und ein lokalisiertes Mikrowellenfeld zur Gewebsbehandlung auszustrahlen.

10. Elektrochirurgische Vorrichtung nach Anspruch 9, wobei der Strahlungsspitzenabschnitt (12, 204) Folgendes umfasst:
eine dielektrische Spitze (214) und
einen distalen leitfähigen Abschnitt des Innenleiters, wobei sich der distale leitfähige Abschnitt der Länge nach in die dielektrische Spitze erstreckt.

11. Elektrochirurgische Vorrichtung nach Anspruch 10, wobei der Außendurchmesser des Koaxialkabels und des Strahlungsspitzenabschnitts gleich oder kleiner 2,5 mm ist.

12. Elektrochirurgische Vorrichtung nach Anspruch 9, wobei der Strahlungsspitzenabschnitt zwei leitfähige Elemente umfasst, die von einem Isolator getrennt sind, und wobei ein leitfähiges Element mit dem Innenleiter des Koaxialkabels verbunden ist und das andere leitfähige Element mit dem Außenleiter des Koaxialkabels verbunden ist.

13. Elektrochirurgische Vorrichtung nach Anspruch 9, wobei der Strahlungsspitzenabschnitt eine Helixantenne umfasst.

14. Elektrochirurgische Vorrichtung nach einem der Ansprüche 9 bis 13, wobei der Strahlungsspitzenabschnitt ausgelegt ist, um als Viertelwellenlänge-Impedanztransformator zu dienen, um eine Eingangsimpedanz an eine Gewebslastimpedanz anzupassen.

## Revendications

1. Appareil électrochirurgical pour traiter un tissu biologique avec de l'énergie de micro-ondes, l'appareil comprenant :
un générateur de signal d'énergie de micro-ondes (102) pour générer une forme d'onde d'énergie de micro-ondes ;
un instrument électrochirurgical (54) agencé pour délivrer la forme d'onde d'énergie de micro-ondes à partir d'une extrémité distale de celui-ci pour un traitement de tissu ;
un dispositif de commande (40) en communication avec le générateur de signal d'énergie de micro-ondes, le générateur de signal d'énergie de micro-ondes étant configuré pour délivrer la forme d'onde d'énergie de micro-ondes sous la forme d'une impulsion de signal d'énergie de micro-ondes, et
le dispositif de commande étant configuré pour commander le profil de l'impulsion de signal d'énergie de micro-ondes pour provoquer une ablation ou une coagulation du tissu biologique et pour empêcher sensiblement l'impulsion de provoquer une accumulation de chaleur dans l'instrument électrochirurgical,
dans lequel le dispositif de commande est configuré pour commander le profil de l'impulsion de telle sorte qu'une puissance de crête de l'impulsion est maintenue à ou au-dessus d'un minimum de puissance de crête qui est réglé pour provoquer une ablation ou une coagulation du tissu biologique pendant l'impulsion de signal d'énergie de micro-ondes, le minimum de puissance de crête étant de 500 W, et dans lequel au moins l'un des éléments suivants s'applique :
(a) le dispositif de commande est configuré pour commander le profil de l'impulsion de sorte qu'une durée d'une partie active de l'impulsion est maintenue à ou en dessous d'une limite de durée de partie active qui est réglée pour empêcher sensiblement la forme d'onde d'énergie de micro-ondes de provoquer un chauffage diélectrique de l'instrument électrochirurgical pendant l'impulsion, la limite de durée de partie active étant de 1 s ;
(b) le dispositif de commande est configuré pour commander le profil de l'impulsion de telle sorte qu'un cycle de service de l'impulsion est maintenu à ou en dessous d'une limite de cycle de service qui est réglée de telle sorte que la chaleur que la forme d'onde d'énergie de micro-ondes provoque pour être accumulée dans l'instrument électrochirurgical pendant une partie active de l'impulsion se dissipe sensiblement pendant une partie inactive de l'impulsion.

2. Appareil électrochirurgical selon la revendication 1, dans lequel le dispositif de commande est configuré pour commander le profil de l'impulsion de telle sorte qu'une énergie de l'impulsion de signal d'énergie de micro-ondes est maintenue à ou au-dessus d'un minimum d'énergie qui est réglé pour provoquer une ablation ou une coagulation du tissu biologique pendant l'impulsion de signal d'énergie de micro-ondes.

3. Appareil électrochirurgical selon la revendication 2, dans lequel le minimum d'énergie est de 1 kJ.

4. Appareil électrochirurgical selon l'une quelconque des revendications précédentes, dans lequel le minimum de puissance de crête est l'un quelconque des suivants : 1 kW, 10 kW, 1 MW, 5 MW.

5. Appareil électrochirurgical selon l'une quelconque des revendications précédentes, dans lequel la limite de durée de partie active est l'une quelconque des suivantes : 0,1 s, 1 ms, 0,2 ms.

6. Appareil électrochirurgical selon l'une quelconque des revendications précédentes, dans lequel au moins l'un des points suivants s'applique :
(a) la limite de cycle de service est de 10 %,
(b) la partie active présente une durée comprise entre 10 µs et 200 µs.

7. Appareil électrochirurgical selon l'une quelconque des revendications 3 à 6 lorsqu'elle dépend de la revendication 2, dans lequel :
le générateur de signal d'énergie de micro-ondes est configuré pour délivrer la forme d'onde d'énergie de micro-ondes sous la forme d'une pluralité d'impulsions de signal d'énergie de micro-ondes, et
le dispositif de commande est configuré pour commander le profil de la pluralité d'impulsions de signal d'énergie de micro-ondes afin de former une pluralité de salves d'impulsions, dans lequel une énergie de chaque salve est maintenue à ou au-dessus du minimum d'énergie.

8. Appareil électrochirurgical selon la revendication 7, dans lequel au moins l'un des points suivants s'applique :
(a) chaque salve présente un cycle de service de salve allant jusqu'à 40 %,
(b) chaque salve présente une durée de partie active de salve allant jusqu'à 200 ms.

9. Appareil électrochirurgical selon l'une quelconque des revendications précédentes, dans lequel l'instrument électrochirurgical comprend :
un câble coaxial pour transporter la forme d'onde d'énergie de micro-ondes, le câble coaxial présentant un conducteur interne, un conducteur externe, et un premier matériau diélectrique (16) séparant le conducteur interne (14) et le conducteur externe (18) ; et
une partie de pointe rayonnante (12, 204) disposée à une extrémité distale du câble coaxial pour recevoir la forme d'onde d'énergie de micro-ondes à partir du câble coaxial et pour rayonner un champ de micro-ondes localisé pour un traitement de tissu.

10. Appareil électrochirurgical selon la revendication 9, dans lequel la partie de pointe rayonnante (12, 204) comprend :
une pointe diélectrique (214), et
une partie conductrice distale du conducteur interne, ladite partie conductrice distale s'étendant longitudinalement dans la pointe diélectrique.

11. Appareil électrochirurgical selon la revendication 10, dans lequel le diamètre extérieur du câble coaxial et de la partie de pointe rayonnante est égal à ou inférieur à 2,5 mm.

12. Appareil électrochirurgical selon la revendication 9, dans lequel la partie de pointe rayonnante comprend deux éléments conducteurs séparés par un isolant, et dans lequel un élément conducteur est connecté au conducteur interne du câble coaxial et l'autre élément conducteur est connecté au conducteur externe du câble coaxial.

13. Appareil électrochirurgical selon la revendication 9, dans lequel la partie de pointe rayonnante comprend une antenne hélicoïdale.

14. Appareil électrochirurgical selon l'une quelconque des revendications 9 à 13, dans lequel la partie de pointe rayonnante est agencée pour agir comme un transformateur d'impédance quart d'onde afin d'adapter une impédance d'entrée à une impédance de charge de tissu.
